# EUROPÄISCHE PATENTSCHRIFT

(11) **EP 1 996 153 B1**
(45) Veröffentlichungstag und Bekanntmachung des Hinweises auf die Patenterteilung: **01.12.2010**
(21) Anmeldenummer: 07703545.9
(22) Anmeldetag: 23.02.2007
(51) Int. Cl.: A61K 8/63, A61Q 17/04, A61Q 19/04

(54) **VERWENDUNG VON GLYCYRRHETINSÄURE UND/ODER GLYCYRRHIZIN ZUR HERSTELLUNG VON ZUBEREITUNGEN ZUR BRÄUNUNG DER HAUT**
USE OF GLYCYRRHETIC ACID AND/OR GLYCYRRHIZIN FOR PRODUCING COMPOSITIONS FOR TANNING THE SKIN
UTILISATION D'ACIDE GLYCYRRHÉTIQUE ET/OU DE GLYCYRRHIZINE POUR LA PRÉPARATION DE COMPOSITIONS POUR LE BRONZAGE DE LA PEAU

(30) Priorität: 01.03.2006 DE 102006009850
(43) Veröffentlichungstag der Anmeldung: 03.12.2008
(73) Patentinhaber: BEIERSDORF AG, 20245 Hamburg (DE)
(72) Erfinder: CERV, Svenja, 22761 Hamburg (DE); GERWAT, Wolfram, 22309 Hamburg (DE); WOLBER, Rainer, 22397 Hamburg (DE); SCHERNER, Cathrin, 22851 Norderstedt (DE)
(74) Vertreter: Wilke, Jochen
(86) Internationale Anmeldenummer: PCT/EP2007/001561
(87) Internationale Veröffentlichungsnummer: WO 2007/101559

(56) Entgegenhaltungen:
- WO-A-01/70188
- WO-A-99/17736
- DE-A1- 10 238 450
- FR-A- 2 777 180
- FR-A1- 2 612 776
- GB-A- 2 257 907
- DATABASE CA [Online] CHEMICAL ABSTRACTS SERVICE, COLUMBUS, OHIO, US; 9. Januar 1988 (1988-01-09), ABE, HIROKO ET AL: "Effects of glycyrrhizin and glycyrrhetinic acid on growth and melanogenesis in cultured B16 melanoma cells" XP002431557 gefunden im STN Database accession no. 1988:298 & EUROPEAN JOURNAL OF CANCER & CLINICAL ONCOLOGY , 23(10), 1549-55 CODEN: EJCODS; ISSN: 0277-5379, 1987, XP002431559
- DATABASE CA [Online] CHEMICAL ABSTRACTS SERVICE, COLUMBUS, OHIO, US; 21. Februar 2005 (2005-02-21), LEE, JONGSUNG ET AL: "Glycyrrhizin induces melanogenesis by elevating a cAMP level in B16 melanoma cells" XP002431558 gefunden im STN Database accession no. 2005:146502 & JOURNAL OF INVESTIGATIVE DERMATOLOGY , 124(2), 405-411 CODEN: JIDEAE; ISSN: 0022-202X, 2005, XP002431560

## Beschreibung

Die vorliegende Erfindung betrifft Zubereitungen zur Bräunung der Haut, insbesondere solche welche gleichzeitig Schutz gegen UV-Strahlung bieten.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

UV-A-Strahlung kann ferner Hautschädigungen hervorrufen, indem das hauteigene Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeiqt, daß offenbar auch Schädigungen der Erbinformationen in den Zellen durch Sonnenlicht, speziell durch UV-A-Strahlung, hervorgerufen werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Die Pigmentierung der menschlichen Haut, wird im wesentlichen durch die Gegenwart von Melanin bewirkt. Melanin und dessen Abbauprodukte (Melanoide), Carotin, Durchblutungsgrad sowie die Beschaffenheit und Dicke des Stratum corneum und andere Hautschichten lassen Hautfarbtöne von praktisch weiß (bei verringerter Füllung oder bei Fehlen der Blutgefäße) oder gelblich über hellbraun-rötlich, bläulich bis braun verschiedener Nuancen und schließlich beinahe schwarz erscheinen. Die einzelnen Hautregionen zeigen aufgrund unterschiedlicher Melanin-Mengen unterschiedliche Tiefe der Farbtönung.

Das natürliche Melanin schützt die Haut vor eindringender UV-Strahlung. Die Anzahl der in den Melanocyten produzierten Melanin-Granula entscheidet über Hell- od. Dunkelhäutigkeit. Bei starker Pigmentierung (z.B. bei Farbigen, aber auch bei Hellhäutigen nach einiger UV-Bestrahlung) ist Melanin auch im Stratum spinosum und sogar im Stratum corneum festzustellen. Es schwächt die UV-Strahlung um bis zu ca. 90%, bevor diese das Corium erreicht.

Je nach Lichtempfindlichkeit werden in der Regel folgende Hauttypen unterschieden:
- Hauttyp I: bräunt nie, bekommt immer einen Sonnenbrand.
- Hauttyp II: bräunt kaum, bekommt leicht einen Sonnenbrand.
- Hauttyp III: bräunt durchschnittlich gut.
- Hauttyp IV: bräunt leicht und anhaltend, bekommt fast nie Sonnenbrand.
- Hauttyp V: dunkle, oft fast schwarze Haut, bekommt nie Sonnenbrand.

Die natürliche Abschirmung der schädlichen UV-Strahlung ist ein handfester Vorteil der natürlichen Hautbräunung. Seit einigen Jahrzehnten gilt darüberhinaus eine "gesunde" Hautfarbe als Zeichen von insbesondere sportlicher Aktivität und wird daher von einer breiten Verbraucherschicht als erstrebenswert erachtet. Vertreter der Hauttypen I und II, die sich einer solchen Hauttönung erfreuen wollen, sind daher ohnehin auf selbstbräunende Präparate angewiesen. Aber auch Vertreter des Hauttyps III, die sich nicht allzusehr den Risiken des Sonnenbades aussetzen und trotzdem gebräunt aussehen wollen, sind dankbare Zielgruppen für selbstbräunende Zubereitungen.

Künstliche Hautbräunung läßt sich auf kosmetischem bzw. medzinischem Wege bewirken, wobei im wesentlichen folgende Ansätze eine Rolle spielen:

Durch regelmäßige Einnahme von Carotin-Präparaten wird Carotin wird im Unterhaut-Fettgewebe gespeichert, die Haut färbt sich allmählich orange bis gelbbraun.

Mit Hilfe abwaschbarer Make-up-Präparate kann eine leichte Hauttönung erzielt werden (z.B. Extrakte aus frischen grünen Walnußschalen, Henna)

Die Anfärbung kann auch auf dem Wege der chemischen Veränderung der Hornschicht der Haut mit sogenannten selbstbräunenden Zubereitungen erfolgen. Wichtigster Wirkstoff ist das Dihydroxyaceton (DHA). Die auf diese Weise erzielte Hautbräunung ist nicht abwaschbar und wird erst mit der normalen Abschuppung der Haut (nach ca. 10-15 Tagen) entfernt. Dihydroxyaceton kann als Ketotriose bezeichnet werden und reagiert als reduzierender Zucker mit den Aminosäuren der Haut bzw. den freien Amino- und Imino-Gruppen des Keratins über eine Reihe von Zwischenstufen im Sinne einer Maillard-Reaktion zu braungefärbten Stoffen, sogenanten Melanoiden, welche gelegentlich auch Melanoidine genannt werden.

Ein Nachteil der Bräunung mit Dihydroxyaceton liegt darin, daß die mit ihm gebräunte Haut im Gegensatze zu "sonnengebräunter" Haut nicht gegen Sonnenbrand geschützt ist.

Die Verwendung von Glycyrrhetinsäure und Glycyrrhizin in Kosmetika und Dermatika ist an sich bekannt. Glycyrrhetinsäure ist durch folgende Struktur gekennzeichnet:

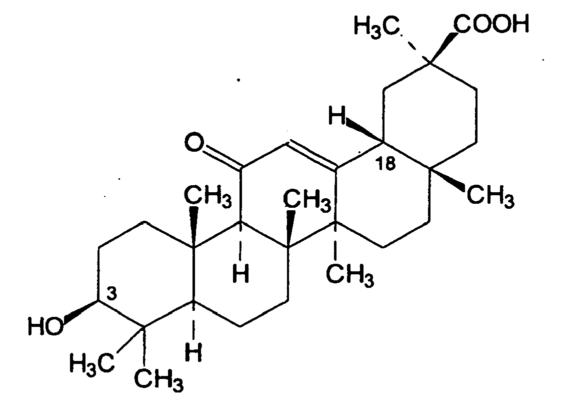

Neben der natürlich vorkommenden 18 β-Form existiert auch eine 18 α-Form. Die aus Süßholz (Glycyrrhiza glabra, Leguminosae) isolierbare Glycyrrhetinsäure ist das Aglycon von Glycyrrhizin (welches das 2 β-Glucuronido-α-glucuronid der Glycyrrhetinsäure darstellt).

Glycyrrhizin ist durch folgende Struktur gekennzeichnet:

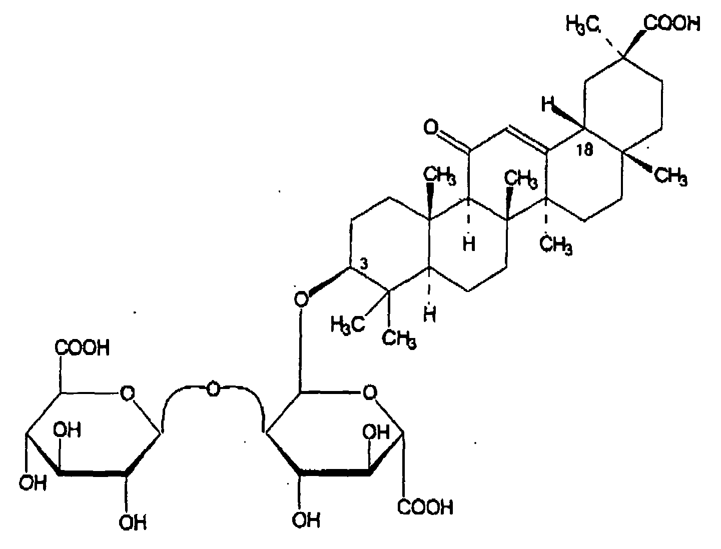

Glycyrrhetinsäure wird als entzündungshemmendes Mittel verwendet. Das Glycyrrhotinsäure-3-Hemisuccinat dient als Ulcustherapeutikum.

Glycyrrhizin wirkt entzündungshemmend und dient in Form von Süßholzsaft (Sirupus liquiritiae) als Hustenmittel und Expektorans.

Glycyrrhetinsäure und Glycyrrhizin sind dem Fachmann als Wirkstoffe mit einer hautaufhellenden Wirksamkeit bekannt.

Den Nachteilen des Standes der Technik abzuhelfen, war also Aufgabe der vorliegenden Erfindung.

Es war indes überraschend und für den Fachmann nicht vorauszusehen, daß die Verwendung von Glycyrrhetinsäure, Glycyrrhizin, geeignet ist zur Herstellung dermatologischer Zubereitungen zur Verstärkung der natürlichen Hautbräunung, **dadurch gekennzeichnet, daß** Glycyrrhetinsäure und/oder Glycyrrhizin in den Zubereitungen als Wirkstoffe zur Hautbräunung eingesetzt werden.

Es war für den Fachmann nicht vorauszusehen gewesen, daß die erfindungsgemäßen Zubereitungen die Pigmentierung der Haut steigern, eine gleichmäßigere Bräunung der Haut ermöglichen, keine unerwünschten Gerüche beim Auftragen auf die Haut entwickeln und eine Verfärbung der Kleidung nach Auftragen auf die Haut vermeiden.

Vorteilhaft enthalten erfindungsgemäße Hautpflegeprodukte 0,0001 - 20 Gewichtsprozente an Glycyrrhetin und/oder Glycyrrhizin .

Bevorzugt enthalten Zubereitungen gemäß der Erfindung 0,001 - 10 Gew.-% an Glycyrrhetin und/oder Glycyrrhizin, bezogen auf die Gesamtzusammensetzung der Zubereitungen.

Ganz besonders bevorzugt enthalten Zubereitungen gemäß der Erfindung 0,01 - 1 Gew.-% an Glycyrrhetin und/oder Glycyrrhizin, bezogen auf die Gesamtzusammensetzung der Zubereitungen.

Erfindungsgemäße Emulsionen im Sinne der vorliegenden Erfindung, z.B. in Form einer Crème, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Die erfindungsgemäßen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Sofern die kosmetische oder dermatologische Zubereitung eine Lösung oder Lotion darstellt, können als Lösungsmittel verwendet werden: Wasser oder wäßrige Lösungen, ferner Öle, wie Triglyceride der Caprin- oder der Caprylsäure, vorzugsweise aber Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, aber auch Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet, so wie bei alkoholischen Lösungsmitteln Wasser ein vorteilhafter weiterer Bestandteil sein kann.

Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung; eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), Öl-in-Wasser-in-Öl (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Die erfindungsgemäßen Wirkstoffe können auch besonders vorteilhaft in Mikroemulsionen, beispielsweise wie in der deutschen Offenlegungsschrift DE-195 9 079 beschrieben, verwendet werden.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoelcosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Diethylhexylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich oder wasserlöslich sein. Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ Zimtsäurederivate, vorzugsweise 2-Ethylhexylmethoxycinnamate (CAS-Nr.: 5466-77-3), welches unter der Handelsbezeichnung Parsol MCX bei der Firma Givaudan erhältlich ist.
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Vorteilhafte wasserlösliche Filtersubstanzen sind z. B.: Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulforisäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bomylidenmethyl)-Benzol und/oder dessen Salze, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Weitere vorteilhaft im Sinne der vorliegenden Erfindung einzusetzende Wirkstoffe sind solche, die den Zustand der Haut positiv beeinflussen, wie insbesondere Wirkstoffe zur positiven Beeinflussung der Altershaut, die die Entstehung von Falten oder auch bestehende Falten vermindern. Vorteilhaft sind insbesondere Biochinone, insbesondere Ubichinon (Q10), Folsäure und ihre Derivate (insbesondere Tetrahydrofolsäure und Dihydrofolsäure), Niacin und seine Derivate (insbesondere Niacinamid), Kreatin und Kreatinin, Carnitin, Biotin, Isoflavon, Hopfen- und Hopfen-Malz-Extrakte. Auch Mittel, die die Restrukturierung des Bindegewebes fördern, wie natürliche und synthetische Isoflavonoide sowie Isoflavonoid-haltige Pflanzenextrakte - wie z. B. Soja- und Klee-Extrakte - können in den erfindungsgemäßen Formulierungen sehr gut verwendet werden. Auch zeigt sich, dass sich die Formulierungen in besonderer Weise eignen, Wirkstoffe zur Unterstützung der Hautfunktionen bei trockener Haut (wie beispielsweise Vitamin C, Biotin, Carnitin, Grüntee-Extrakte, Eucalyptusöl, Harnstoff und Mineralsalze (wie z. B. NaCl, Meeresmineralien) sowie Osmolyte (wie z. B. Inositol, Betain, quartäre Ammoniumverbindungen)) zu verwenden. In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süßholzes, Licochalcone, insbesondere Licochalcon A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxyaenase, aber auch des 5-Lipoxvgenase Inhibitor Proteins, FLAP. Die zuvor genannten Wirkstoffe sind ferner insbesondere geeignet, um ein (Energie-) Depot anzulegen oder zu erneuern sowie die Reparatur (Repair) unterschiedlicher zellulärer Strukturen, insbesondere der DNA zu aktivieren.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und dermatologische Anwendungen geeigneten oder gebräuchlichen Antioxidantien verwendet werden.

Ganz besonders vorteilhaft im Sinne der vorliegenden Erfindung können wasserlösliche Antioxidantien eingesetzt werden, wie beispielsweise Vitamine, z. B. Ascorbinsäure und deren Derivate - insbesondere Ascorbylpalmitat, Na- und Mg-Ascorbylphosphat und Ascorbylacetat - sowie Rutinsäure und deren Derivate insbesondere alpha Glucosylrutin, Quercetin und Isoquercetin.

Besonders bevorzugte Antioxidantien sind ferner Vitamin E und dessen Derivate (insbesondere Vitamin E-Acetat), Vitamin A und dessen Derivate (insbesondere Vitamin-A-Palmitat) sowie Carnosin, Butylhydroxytoluol, Butylhydroxyanisol, beta-Alanin sowie Carotinoide (insbesondere beta-Carotin) und Phytoen.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Die Zubereitungen gemäß der vorliegenden Erfindung können ferner vorteilhaft auch Selbstbräunungssubstanzen wie beispielsweise Dihydroxyacteon, Melaninderivate und Erythrulose enthalten, wobei diese Substanzen in Konzentrationen von 0,1 Gew.-% bis zu 8 Gew.-%, bevorzugt 0,4 - 2 Gew.-% bezogen auf das Gesamtgewicht der Zubereitung eingesetzt werden. Die erfindungsgemäßen Formulierungen können ferner vorteilhaft neben Dihydroxyaceton auch Nussextrakte enthalten sowie weitere Substanzen, welche die Bräune erhalten oder erzeugen oder zusätzlich verstärken sollen. Als extrem vorteilhaft hat sich die Kombination von Selbstbräunern, insbesondere Dihydroxyaceton und Erythrulose, mit den erfindungsgemäßen Verbindungen in kosmetischen Zubereitungen erwiesen, um unerwünschte Hyperpigmentierungen (Melasma, Altersflecken, Sommersprossen etc.) zu behandeln.

Die folgenden Beispiele sollen die Verkörperungen der vorliegenden Erfindungen verdeutlichen.

### Lotion

| | |
|---|---|
| Wasser | ad 100 |
| Stearinsäure | 2 |
| Dicaprylylether | 3 |
| Octyldodecanol | 2 |
| C12-15 Alkylbenzoate | 4 |
| Cetylalkohol | 2 |
| Cetearyll Ethylhexanoat + Isopropylmyristat | 2 |
| Glycerin | 5 |
| Ethylhexyl Methoxycinnamat | 2 |
| Ti02 | 1 |
| Cetylpalmitat | 1 |
| Glyceryl Stearat | 1 |
| Phenoxyethanol | 0.4 |
| Butyl Methoxydibenzoxylmethan | 2 |
| Parfum | q.s. |
| EDTA | 0.2 |
| Carbomer | 0.2 |
| Magnesium Aluminium Silicar | 0.2 |
| Methylparaben | 0.1 |
| Vitamin E Acetat | 0.1 |
| Glycyrrhetinsäure | 0.1 |
| Propylparaben | 0.05 |
| BHT | 0.05 |
| DHA | 1 |

### Nachtcreme:

| | **Gew.-%** |
|---|---|
| Glycerylstearatcitrat | 2 |
| Cetylricinoleat | 1 |
| Stearylalkohol | 2 |
| Cetylalkohol | 2 |
| Hydrierte Kokosfettglyceride (Hydrogenated Coco Glycerides) | 1 |
| Caprylsäure/Caprinsäuretriglyceride | 3 |
| Ethylhexylkokosfettsäureester | 2 |
| Dicaprylylether | 2 |
| C12-15 Alkylbenzoate | 3 |
| Tocopherylacetat | 1 |
| Ubichinon (Coenzym Q10) | 0,05 |
| Natriumascorbylphosphat | 0,1 |
| Glycyrrhetinsäure | 0.1 |
| p-Hydroxybenzoesäurealkylester (Paraben) | 0,4 |
| Methylpropandiol | 1 |
| Carrageenan | 0,1 |
| Carbomer | 0,2 |
| EDTA | 0,2 |
| Glycerin | 7 |
| wasser- und/oder öllösliche Farbstoffe | 0,05 |
| Füllstoffe/ Additive (Nylon, BHT) | 0,5 |
| Parfüm | q.s. |
| Wasser | ad 100 |

### Tagescreme

| | **Gew.-%** |
|---|---|
| PEG-40-Stearat | 1 |
| Glycerylstearat | 3 |
| Cetearylalkohol | 2 |
| Dimeticone | 1 |
| Hydrierte Kokosfettglyceride (Hydrogenated Coco Glycerides) | 2 |
| Caprylsäure/Caprinsäuretriglyceride | 2 |
| Octyldodecanol | 2 |
| Dicaprylylcarbonat | 2 |
| C12-15 Alkylbenzoate | 3 |
| Ethylhexylmethoxycinnamat | 4 |
| Butylmethoxydibenzoylmethan | 2 |
| Tocopherylacetat | 1 |
| Panthenol | 0,5 |
| Natriumascorbylphosphat | 0,1 |
| Glycyrrhetinsäure | 0.1 |
| p-Hydroxybenzoesäurealkylester (Paraben) | 0,4 |
| Methylpropandiol | 1 |
| Carbomer | 0,2 |
| Xanthan Gum | 0,1 |
| EDTA | 0,2 |
| Glycerin | 8 |
| Tapiocastärke | 0,05 |
| Füllstoffe/ Additive , | 0,3 |
| Parfüm | q.s. |
| Wasser | ad 100 |

### Gesichtscreme

| | |
|---|---|
| Polyglyceryl-3-Methylglucosedistearat | 2 |
| Sorbitanstearat | 3 |
| Cetylalkohol | 2 |
| Myristylmyristate | 1 |
| Dicaprylylether | 3 |
| Octyldodecanol | 2 |
| C12-15 Alkylbenzoate | 3 |
| Cetearyll Ethylhexanoat + Isopropylmyristat | 2 |
| Ethylhexyl Methoxycinnamat | 2 |
| Ethylhexyltriazon | 1 |
| Butyl Methoxydibenzoxylmethan | 2 |
| Magnesium Aluminium Silicar | 0.2 |
| Glycerin | 5 |
| Phenoxyethanol | 0.4 |
| Parabene | 0.3 |
| Vitamin E Acetat | 0.1 |
| Glycyrrhetinsäure | 0.1 |
| BHT | 0.05 |
| EDTA | 0.2 |
| Carbomer | 0.2 |
| Parfüm | q.s. |
| Wasser | ad 100 |

## Patentansprüche

1. Verwendung von Glycyrrhetinsäure und/oder Glycyrrhizin zur Herstellung dermatologischer Zubereitungen zur Verstärkung der natürlichen Hautbräunung, **dadurch gekennzeichnet, daß** Glycyrrhetinsäure und/oder Glycyrrhizin in den Zubereitungen als Wirkstoffe zur Hautbräunung eingesetzt werden.

2. Verwendung nach Anspruch 1, **dadurch gekennzeichnet, daß** die Zubereitungen 0,0001 - 20 Gew.-% Glycyrrhetinsäure und/oder Glycyrrhizin, bezogen auf die Gesamtzusammensetzung der Zubereitungen, enthalten.

3. Verwendung nach Anspruch 1, **dadurch gekennzeichnet, daß** die Zubereitungen 0,001 - 10 Gew.-% an Glycyrrhetinsäure und/oder Glycyrrhizin, bezogen auf die Gesamtzusammensetzung der Zubereitungen, enthalten.

4. Verwendung nach Anspruch 1 , **dadurch gekennzeichnet, daß** die Zubereitungen 0,01 - 1 Gew.-% an Glycyrrhetinsäure und/oder Glycyrrhizin, bezogen auf die Gesamtzusammensetzung der Zubereitungen, enthalten.

## Claims

1. Use of glycyrrhetic acid and/or glycyrrhizin for producing dermatological preparations for enhancing natural skin tanning, **characterized in that** glycyrrhetic acid and/or glycyrrhizin are used in the preparations as skin tanning active ingredients.

2. Use according to Claim 1, **characterized in that** the preparations comprise 0.0001-20% by weight of glycyrrhetic acid and/or glycyrrhizin, based on the total composition of the preparations.

3. Use according to Claim 1, **characterized in that** the preparations comprise 0.001-10% by weight of glycyrrhetic acid and/or glycyrrhizin, based on the total composition of the preparations.

4. Use according to Claim 1, **characterized in that** the preparations comprise 0.01-1% by weight of glycyrrhetic acid and/or glycyrrhizin, based on the total composition of the preparations.

## Revendications

1. Utilisation de l'acide glycyrrhétinique et/ou de la glycyrrhizine pour la préparation de compositions dermatologiques pour le renforcement du bronzage naturel de la peau, **caractérisée en ce que** l'acide glycyrrhétinique et/ou la glycyrrhizine sont utilisés dans les compositions comme substances actives pour le bronzage de la peau.

2. Utilisation selon la revendication 1, **caractérisée en ce que** les compositions contiennent 0,0001-20% en poids d'acide glycyrrhétinique et/ou de glycyrrhizine, par rapport à la composition totale des compositions.

3. Utilisation selon la revendication 1, **caractérisée en ce que** les compositions contiennent 0,001-10% en poids d'acide glycyrrhétinique et/ou de glycyrrhizine, par rapport à la composition totale des compositions.

4. Utilisation selon la revendication 1, **caractérisée en ce que** les compositions contiennent 0,01-1% en poids d'acide glycyrrhétinique et/ou de glycyrrhizine, par rapport à la composition totale des compositions.
